# EUROPEAN PATENT APPLICATION

(11) **EP 1 614 679 A1**
(43) Date of publication of application: **11.01.2006**
(21) Application number: 05013524.3
(22) Date of filing: 23.06.2005
(51) Int. Cl.: C07C 391/00, C01B 19/00, A61K 33/04

(54) **Methylselenol zinc salt and compositions containing it**

(30) Priority: 09.07.2004 IT MI20041374
(71) Applicant: MARFARMA HOLDING S.p.A., 20154 Milano (IT)
(72) Inventor: Heyda, Alessandro c/o Marfarma Holding S.p.A., 20154 Milano (IT)
(74) Representative: Bianchetti, Giuseppe

(57) **Abstract**

Methylselenol zinc salt, of formula (CH₃Se)₂Zn, obtainable by reaction between zinc chloride and methylselenol sodium salt. The novel zinc salt of the invention can be advantageously used as a low-dosage component of pharmaceutical, nutraceutical and dietetic compositions, as an agent able to prevent the onset of neoplasias.

## Description

### Disclosure

The present invention relates to the methylselenol zinc salt and compositions containing it.

Selenium, and particularly some organic compounds thereof, is known to play a fundamental role in a number of biochemical processes.

In addition to a general antioxidizing activity, selenium compounds have recently been investigated for their chemopreventive activity against neoplasias.

By way of example, Ip et al, in *Cancer Res.* 60, 2882-2886, 2000, have investigated the *in vitro* and *in vivo* activity of methylseleninic acid and proved that a monomethylated metabolite of selenium is an important chemoprotective factor.

According to the extensive studies carried out by the same researchers, methylselenol (CH₃SeH), deriving from metabolic transformations of the selenide and selenomethionine, is a remarkably effective compound for the prevention of tumors (*Cancer Res. 50,* 1206-1211, 1990; *ibidem,* 51 595-600,1991).

On the other hand, methylselenol is an extremely volatile liquid, with boiling point of 25°C, and is therefore difficult to use as such in the formulation of pharmaceutical and nutraceutical compositions or dietary supplements.

It has now been found that the methylselenol zinc salt, besides being an easy-to-handle solid, has improved functional characteristics which make it particularly suitable for the preparation of the above cited pharmaceutical and nutraceutical compositions or dietary supplements, in combination with suitable carriers and excipients and optionally with other active ingredients.

Said compositions are a further object of the invention.

The methylselenol zinc salt, that can be represented by the following formula:

(CH₃Se)₂Zn

can be prepared by reacting methylselenol sodium salt with a zinc salt, preferably with zinc chloride in stoichiometric amounts, in water. Methylselenol sodium salt is conveniently obtained by reduction of the dimethylselenide and subsequent reaction with stoichiometric amounts of sodium hydroxide. The resulting salt can be freeze-dried or precipitated by addition of a non-solvent and/or by concentration and cooling. Alternatively, a dispersion of the salt of the invention in zinc carbonate, having a content in the salt of the invention approximately ranging from 0.1 to 5%, can be prepared. In fact, in view of the very low dosages necessary for the compositions containing the methylselenol zinc salt, the use of a dispersion of selenol salt in an inert matrix, such as zinc carbonate, can be more convenient, in that it can act both as a zinc ions carrier and a pH control agent.

The methylselenol zinc salt is a white powder, highly hygroscopic, with characteristic garlic odour.

The doses active for the prevention are of the order of the micrograms. The preferred administration route is of course the oral one, considering the characteristics of the product and the use for long-term preventive treatments. However, other administration routes are not excluded. Furthermore, in some cases also a therapeutical treatment, optionally in combination with other chemotherapeutics, can be envisaged.

Examples of suitable compositions which can be administered through the oral route are soft- or hard- gelatin capsules, tablets, bars, granulates in sachets, syrups, chewing-gums and the like.

The compositions of the invention will further contain other compounds known to have chemopreventive and/or antioxidant activity. Examples of said compounds comprise vitamin C, vitamin E, lycopene, beta-carotene, lutein, resveratrol, sulforafan, vegetable extracts, such as garlic, broccoli and other brassicacee extracts, soy and red clover extracts.

## Claims

1. Methylselenol zinc salt, of formula:
(CH₃Se)₂Zn.

2. Compositions containing the salt of claim 1 in admixture with suitable carriers.

3. The use of the methylselenol zinc salt for the preparation of compositions with chemopreventive and chemotherapeutic activity.
